# EUROPEAN PATENT APPLICATION

(11) **EP 2 724 679 A1**
(43) Date of publication of application: **30.04.2014**
(21) Application number: 13189893.4
(22) Date of filing: 23.10.2013
(51) Int. Cl.: A61B 17/70

(54) **Transverse connector and related methods**

(30) Priority: 23.10.2012 US 201261717296 P
(71) Applicant: Nexus Spine, L.L.C., Salt Lake City, UT 84121 (US)
(72) Inventor: Hawkes, David T., Salt Lake City, UT 84121 (US)
(74) Representative: Adam, Holger

(57) **Abstract**

A connecting system (10) includes a longitudinal connector (12) having two opposite ends and at least one fastener (30), configured to couple at least one of the ends of the longitudinal connector to a patient's anatomy. At least one clamping assembly (20, 22) is operable to secure the longitudinal connector to the fastener. The clamping assembly allows adjustment of relative positions of the longitudinal connector and the fastener prior to fixing relative positions of the longitudinal connector and the fastener.

## Description

### PRIORITY CLAIM

Priority is claimed to co-pending U.S. Provisional Patent Application Serial No. 61/717,296, filed October 23, 2012, which is hereby incorporated herein by reference in its entirety.

### BACKGROUND OF THE INVENTION

### Field of the Invention

The present invention relates generally to the field of coupling systems for use in surgical implants. More particularly, the present invention relates to such systems for use in transversely connecting components of surgical implants. The present system is well suited for use in spinal implant procedures.

### SUMMARY OF THE INVENTION

In accordance with one aspect of the invention, a connecting system is provided, including a longitudinal connector having two opposite ends and at least one fastener. The fastener can be configured to couple at least one of the ends of the longitudinal connector to a patient's anatomy. At least one clamping assembly can be operable to secure the longitudinal connector to the fastener. The clamping assembly can allow adjustment of relative positions of the longitudinal connector and the fastener prior to fixing relative positions of the longitudinal connector and the fastener by installing the fastener.

In accordance with another aspect of the invention, a method of securing relative position of two components of a patient's anatomy is provided, including: positioning opposite first and second ends of a longitudinal connector such that the opposite ends of the longitudinal connector span a distance between two components of the patient's anatomy; positioning a first clamp assembly affixed to the first end of the longitudinal connector; coupling the first clamp assembly to a first component of the patient's anatomy by inserting a first fastener through the first clamp assembly; adjusting relative positions of the longitudinal connector and the first clamp assembly; fixing the first clamp assembly to the first component of the patient's anatomy to thereby fix relative positions of the longitudinal connector and the first clamp assembly; and securing the second end of the longitudinal connector to a second component of the patient's anatomy.

In accordance with another aspect of the invention, an anatomical bracing device for securing relative positions of a patient's anatomy is provided, including a longitudinal connector having a proximal end and at least one distal end. At least two clamp assemblies can be affixed to each end of the longitudinal connector. At least one fastener can extend through each of the clamp assemblies. The bracing device can be configured such that securing the fasteners to the patient's anatomy both secures the longitudinal connector to the patient's anatomy and fixes relative positions of the longitudinal connector and clamp assemblies relative to each other.

Additional features and advantages of the invention will be apparent from the detailed description which follows, taken in conjunction with the accompanying drawings, which together illustrate, by way of example, features of the invention.

### BRIEF DESCRIPTION OF THE DRAWINGS

The following drawings illustrate exemplary embodiments for carrying out the invention. Like reference numerals refer to like parts in different views or embodiments of the present invention in the drawings.
FIG. 1 is a perspective view of an assembled transverse connecting system in accordance with an embodiment of the invention;
FIG. 2 includes an exploded perspective view of the connecting system of FIG. 1;
FIGs. 3A, 3B and 3C show various views of a fastener for use with the connecting system of FIG. 1;
FIGs. 4A, 4B, 4C and 4E show various views of a clamping system for use with connecting system of FIG. 1; and
FIGs. 5A, 5B and 5C show various views of the longitudinal connector for use with the connecting system of FIG. 1; and
FIG. 6 depicts a flow chart showing a method of implementing the connecting system of the present invention in a surgical procedure.

### DETAILED DESCRIPTION

Reference will now be made to the exemplary embodiments illustrated in the drawings, and specific language will be used herein to describe the same. It will nevertheless be understood that no limitation of the scope of the invention is thereby intended. Alterations and further modifications of the inventive features illustrated herein, and additional applications of the principles of the inventions as illustrated herein, which would occur to one skilled in the relevant art and having possession of this disclosure, are to be considered within the scope of the invention.

### Definitions

As used herein, the singular forms "a" and "the" can include plural referents unless the context clearly dictates otherwise. Thus, for example, reference to a "fastener" can include one or more of such fasteners.

As used herein, the term "substantially" refers to the complete or nearly complete extent or degree of an action, characteristic, property, state, structure, item, or result. As an arbitrary example, an object that is "substantially" enclosed would mean that the object is either completely enclosed or nearly completely enclosed. The exact allowable degree of deviation from absolute completeness may in some cases depend on the specific context. However, generally speaking the nearness of completion will be so as to have the same overall result as if absolute and total completion were obtained.

The use of "substantially" is equally applicable when used in a negative connotation to refer to the complete or near complete lack of an action, characteristic, property, state, structure, item, or result. As another arbitrary example, a composition that is "substantially free of" an ingredient or element may still actually contain such item as long as there is no measurable effect thereof.

As used herein, the terms "attached," "coupled," fixed," etc., can be used to describe a condition in which two or more components are coupled to one another in such a manner that they function as intended: that is, the force required to uncouple the components is sufficiently large such that the components will remain attached to one another during the service for which they were designed. Unless indicated to the contrary, such "coupled" components can be separable if sufficient force is applied to the components. In some aspects of the invention, components are elastically fixed or coupled to one another and will remain fixed during the useful life of the product for which they are designed; however, they may be uncoupled from one another using an appropriate level of force (applied in an appropriate manner and location), and will return to an original configuration (e.g., a condition, state, shape, size, etc.), which existed prior to the components being coupled to one another.

As used herein, the term "about" is used to provide flexibility to a numerical range endpoint by providing that a given value may be "a little above" or "a little below" the endpoint.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

Numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 to about 5" should be interpreted to include not only the explicitly recited values of about 1 to about 5, but also include individual values and sub-ranges within the indicated range. Thus, included in this numerical range are individual values such as 2, 3, and 4 and sub-ranges such as from 1-3, from 2-4, and from 3-5, etc., as well as 1, 2, 3, 4, and 5, individually.

This same principle applies to ranges reciting only one numerical value as a minimum or a maximum. Furthermore, such an interpretation should apply regardless of the breadth of the range or the characteristics being described.

### Invention

The present invention relates generally to connecting system for use in surgical procedures. While the system can be utilized in a variety of procedures, it is well suited for use in spinal implant procedures. In particular, the present invention is particularly suitable for spinal fusions wherein the relative positions of two vertebrae are secured with respect to one another.

The present invention provides a low cost, low profile, and simple to use transverse connector for use in applications such as spinal fusion surgery. This is in contrast to conventional systems, which have typically been either i) low cost and bulky, or ii) high cost and simple to use. The present system can also be implanted via minimally invasive surgical approaches, which typically do not provide a large midline access to the surgical site. Minimally invasive surgical approaches typically only provide two small access ports at each side of the spine, rather than a single, large central access. The present system can be utilized in such procedures because it provides a transverse connector that can be preassembled with polyaxial, frictional interfaces.

The present invention is inexpensive to manufacture, extremely low bulk and provides six degrees of freedom for simplified use. This functionality is provided with only two points of locking, which are conveniently located at the ends of the connector. Thus, the system is optimized for a minimally invasive surgical approach.

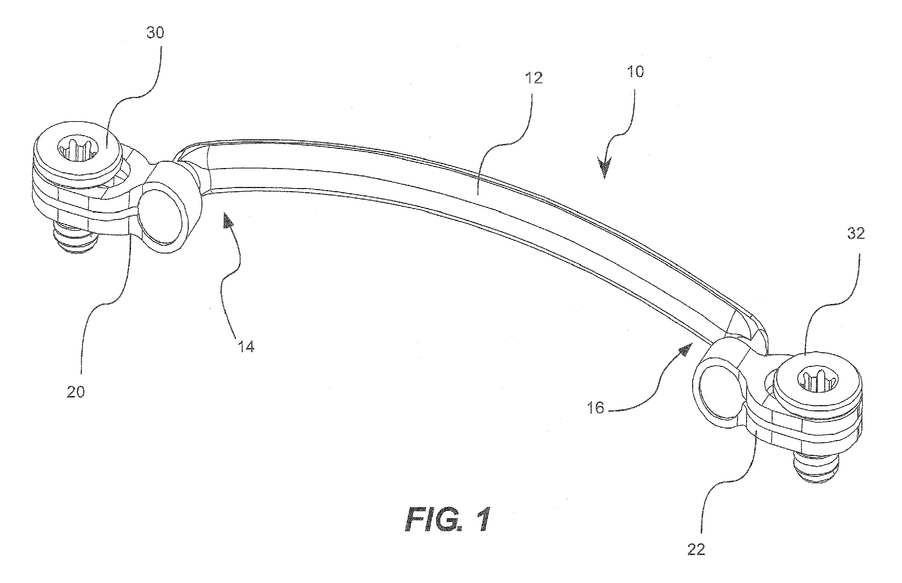

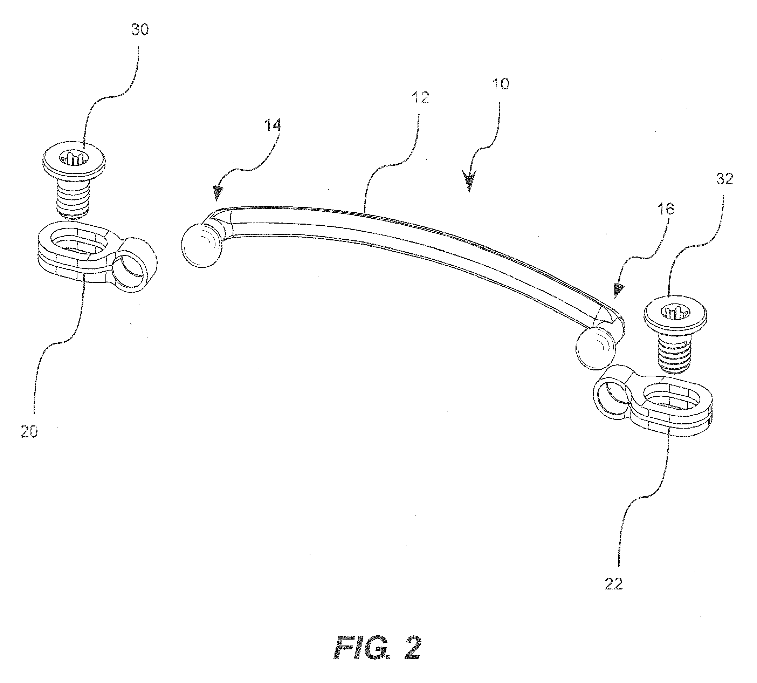

A connecting system suitable for such spinal implant procedures is shown throughout the various figures. With reference to FIGs. 1-2, a connecting system in accordance with the present invention is shown generally at 10. The connecting system 10 includes a longitudinal connector 12 which may be referred to herein as a cross or span bar 12. The longitudinal connector has a main body with two distal ends. The distal ends of the longitudinal connector can be provided with respective clamp or clamping assemblies 20 and 22 as well as respective fasteners 30 and 32.

The connecting system of the example embodiment can include a first fastener 30 designed to be attached to the head of a pedicle screw which is attached to a first part of a patient's anatomy (for example, a vertebrae within the patient's spinal column). The longitudinal connector 12 can span a distance between this first part of the patient's anatomy to a second part of the patient's anatomy having a similar pedicle screw (for example, a second vertebra within the patient's spinal column). In another example, the technology can be attached to thoracic hooks or rod-to-rod connectors, which are commonly used in the treatment of the thoracic spine. In particular reference to the use of the connecting system with a patient's spinal column, the longitudinal connector 12 can be placed substantially close to the patient's spine and thereby provides a low profile so as to provide decreased soft tissue obstruction when compared to prior art systems.

The u-shape of the device allows circumnavigation of the spinous processes, so as to decrease the need to violate posterior elements of the spine.

Numerous features and configurations may be discussed below in relation to clamping assembly 20 and its respective fastener 30 and distal end 14. However, it should be noted that each feature and configuration discussed with respect to these components are similarly applicable to clamping assembly 22 and its respective fastener 32 and distal end 16, in any combination. Thus, the following discussion does not necessarily provide "mirrored" discussions of each of the clamp assemblies.

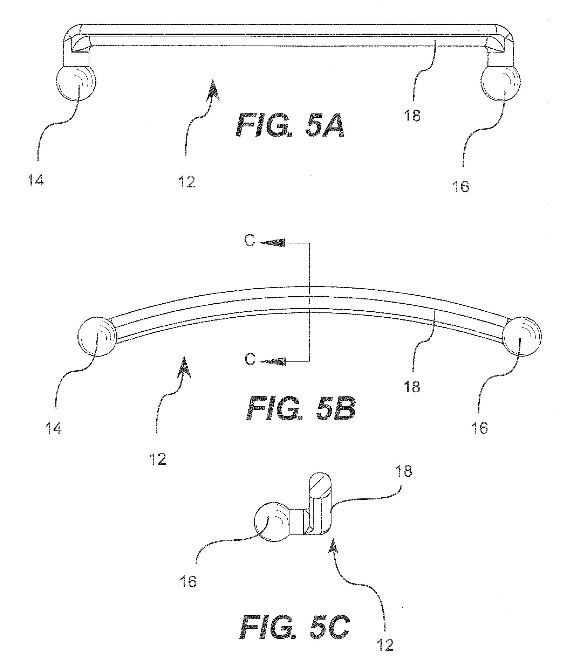

The clamping assemblies 20, 22, as discussed above, can be configured to attach to distal ends 14, 16 of the longitudinal connector 12. The distal end 14 of the longitudinal connector may be provided as a semi-spherical ball or ball joint (best appreciated in FIG. 5A and 5B) which may provide a multi-axial connection between the clamping assembly 20 and the longitudinal connector 12. This allows the distal end 14 of the longitudinal connector to rotate within the clamping assembly 20 and essentially the longitudinal connector 12 can extend from the clamping assembly 20 in any direction. In this manner, the system provides at least a first degree of freedom to the assembly 10. Upon insertion of the fastener 30, 32, and the tightening thereof, the clamping assembly 20, 22 tightens and secures or locks the relative positions of the fastener 30, the clamping assembly 20 and the longitudinal connector 12.

This arrangement provides a number of advantages. For example, tightening of the fastener 30 imposes little or no deflective stresses or rotational moments which may cause shifting of the relative positions of the longitudinal connector 12 and the clamping assembly 20 upon tightening of the fastener 30. Additionally, the fastener 30 provides a progressively increasing clamping force to the clamping assembly 20 as it is progressively tightened. In other words, the fastener 30 may be partially tightened to provide a partial clamping force providing some resistance between the relative positions of the clamping assembly 20 and the longitudinal connector 12. In this condition, the clamp assembly 20 may be capable of retaining a position but may still be manually manipulated by the surgeon prior to full tightening of the assembly and locking of the relative positions. Once locked into position, the various components cannot be easily moved relative to one another.

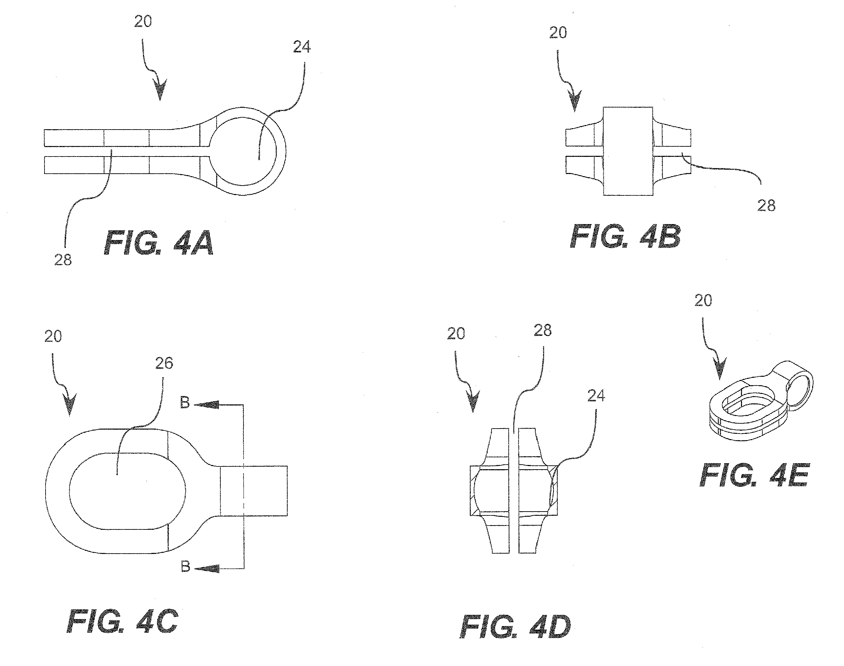

In order to ensure a strong lock of the relative positions, the clamping assembly 20 may be provided with a semi-spherical socket (24 in FIGs. 4A, 4D, etc.) having a textured inner surface designed to mate with a textured outer surface of a semi-spherical ball provided as the distal end 14 of the longitudinal connector 12. In this manner, the two textured surfaces may be configured such that they "bite" into one another and provide an extremely strong interference or frictional fit once the fastener has been tightened.

Another aspect of the present invention resides in the use of a saddle clamp as the clamping assembly 20. The saddle clamp can include a U-shaped body having a slot 28 (FIG. 4D). By inserting the fastener 30 through fastener hole 26, then tightening the fastener 30, a compression force may be applied to constrict the slot 28 and thereby conform and constrict the semi-spherical socket 24 around the distal end 14 of the longitudinal connector 12. Another advantage may be realized by providing an elongated fastener hole 26. By providing an elongated fastener hole 26, the clamping assembly 20 provides the ability to translate (longitudinally) prior to tightening of the fastener 30. In this manner, the entire assembly is provided with an additional degree of freedom for proper positioning prior to locking or securing the assembly in place.

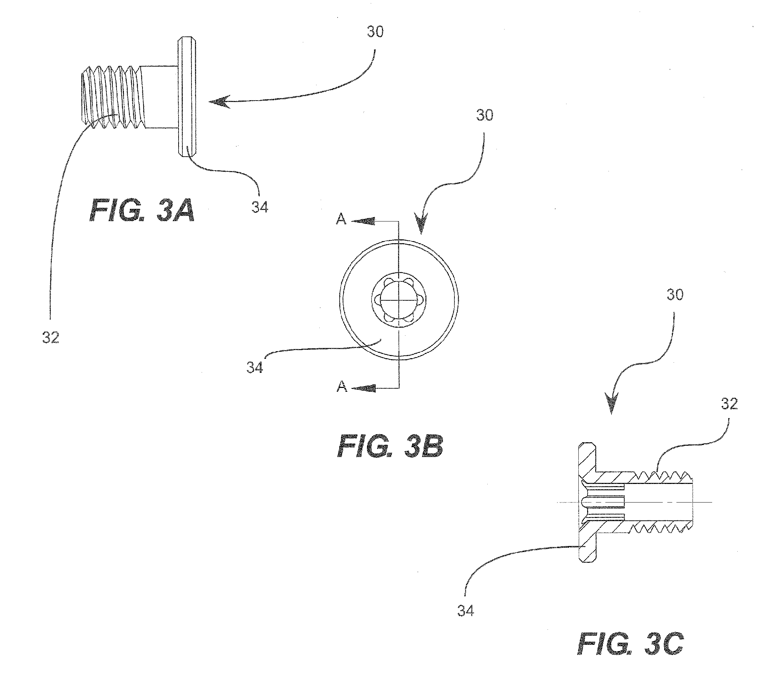

The fastener 30 can include a threaded portion 32 (FIGs. 3A-3C) configured to thread into a head portion of a pedicle screw (not shown). A flange portion 34 can be configured to provide a clamping force to the saddle clamp 20. By threading the threaded portion 32 into the head portion of the pedicle screw (or another suitable substrate), the flange portion 34 is drawn toward the head portion of the pedicle screw and creates a compression force. This compression force not only locks the saddle clamp in position longitudinally, it causes the semi-spherical socket 24 to constrict about the ball, preventing rotational movement. It should be appreciated that the fastener 30 may also be provided as a pedicle screw itself and that the clamping force may be provided between a similar flange on the fastener and either the substrate or against a locking nut provide along the threaded portion 32.

The fastener 30 can be configured to be installed within a pedicle screw that is already installed within a patient's anatomy (e.g., within a component of the patient's spine). It is to be understood, however, that the fastener can be coupled to the patient's anatomy in a variety of manners. As discussed above, the fastener can itself be a pedicle screw. It should further be appreciated that in other embodiments, the fastener can be attached to other components that form a part of the spinal implant: for example, the fastener can be attached to one or more connecting rods that connect spinal components or spinal implant components. Thus, the fastener can, in some embodiments, be attached in a location offset from the location of the pedicle screw.

As shown in FIGs. 5A, 5B and 5C, the longitudinal connector 12 can be formed having a longitudinal or elongated body 18, with distal ends 14 and 16 extending away from the elongated body at approximately 90 degrees to form the semi-spherical ball joints which mate with their respective clamping assemblies. This configuration allows the elongated body 18 to be displaced away from the effective line of action of the connector assembly such that it does not restrict access to the area being immobilized by the connector. For example, this offset allows the surgeon to operate on the injured vertebrae being fused by the connector assembly after installation.

Additionally, the longitudinal body 18 of the longitudinal connector may further be shaped in an arc to curve or extend away from the intervening vertebrae or portion of the patient's body. This arc gives even greater ability to access the intervening portion of the patient's body for subsequent procedures. Alternatively, the longitudinal body may be shaped in a custom configuration such that it conforms more closely to the patient's anatomy giving it a lower profile for greater comfort in the event of extended or long term installations.

Further, the cross bar 18 can alternatively be configured to attach directly above the pedicle screw, which can simplify minimally invasive implantation and may better fit within the constraints of the anatomy. As all locking of the transverse connector is affected at the pedicle screw rather than at the midline, a myriad of configurations can be achieved.

In addition to the structure discussed and shown herein, the present invention also provides methods of manufacturing and implanting various transverse connectors utilizing the structural and functional capability of the system outlined herein.

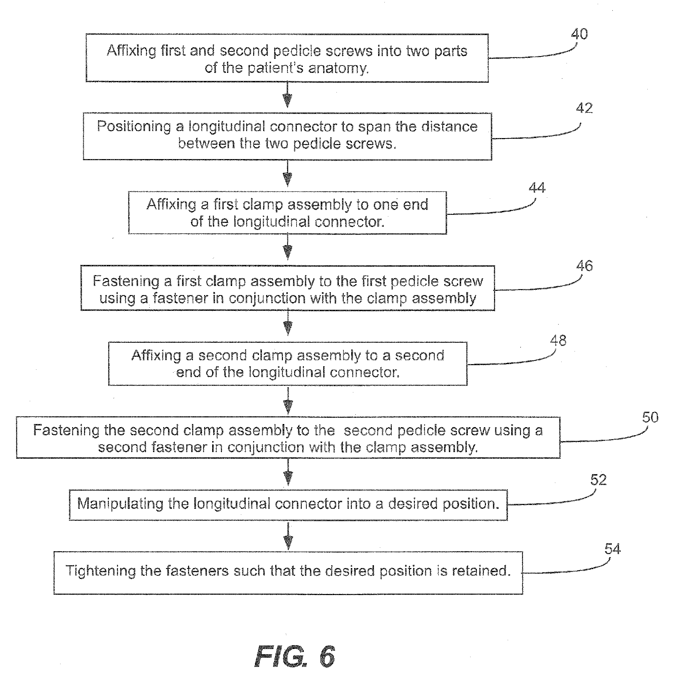

With reference to FIG. 6, in one example, a method for using the connecting system for securing the relative positions of two parts of a patient's anatomy is shown. In particular, a first step 40 can include affixing a first and a second pedicle screw into two separate portions of the patient's anatomy. A second step 42 can involve positioning a longitudinal connector to span the distance between the first and second pedicle screws located in the two parts of the patient's anatomy. A third step 44 can include affixing a first clamp assembly to one end of the longitudinal connector followed by a fourth step 46 which can include fastening of the first clamp assembly to the first pedicle screw using a fastener in conjunction with the clamp assembly.

It should be appreciated that the fastener may itself be the first pedicle screw in which case a separate fastener may not be required. A fifth step 48 can involve affixing a second clamp assembly to a second end of the longitudinal connector. A sixth step can include fastening the second clamp assembly to the second pedicle screw using a second fastener in conjunction with the claim assembly. Similarly, the second fastener may also itself be a pedicle screw in which case a separate fastener may not be required to fasten the second clamp assembly to the second pedicle screw. The fasteners can then be partially tightened and a sixth step 52 can involve manipulating the longitudinal connector into a desired position and finally a seventh step 54 can involve tightening the fasteners such that the desired relative position of each component is retained.

It should be appreciated that the two parts of the patient's anatomy discussed above may be two separate vertebrae of the patient's spinal column as applied to a spinal procedure, i.e. a spinal fusion. In addition, the two parts or components of the patient's anatomy can be a single anatomical feature such as a native skeletal structure that has been separated into two segments (e.g., a broken bone).

It is to be understood that the above-referenced arrangements are illustrative of the application for the principles of the present invention. Numerous modifications and alternative arrangements can be devised without departing from the spirit and scope of the present invention while the present invention has been shown in the drawings and described above in connection with the exemplary embodiments(s) of the invention. It will be apparent to those of ordinary skill in the art that numerous modifications can be made without departing from the principles and concepts of the invention as set forth in the examples.

## Claims

1. A connecting system, comprising:
a longitudinal connector having two opposite ends;
at least one fastener, configured to couple at least one of the ends of the longitudinal connector to a patient's anatomy; and
at least one clamping assembly, operable to secure the longitudinal connector to the fastener; wherein
the clamping assembly allows adjustment of relative positions of the longitudinal connector and the fastener prior to fixing relative positions of the longitudinal connector and the fastener by installing the fastener.

2. The system of claim 1, wherein the fastener comprises a pedicle screw.

3. The system of claim 1, wherein the fastener includes a secondary interface configured to be secured to a pedicle screw.

4. The system of claim 3, wherein the fastener is installable within the pedicle screw along a common longitudinal axis.

5. The system of claim 1, wherein the clamping assembly allows translational adjustment of relative positions of the longitudinal connector and the fastener prior to fixing relative translational positions of the longitudinal connector and the fastener.

6. The system of claim 1, wherein the clamping mechanism includes a saddle clamp.

7. The system of claim 6, wherein the saddle clamp and the longitudinal connector are coupled with a ball joint, and wherein tightening the saddle clamp constrains a ball within the ball joint.

8. A method of securing relative position of two components of a patient's anatomy, comprising:
positioning opposite first and second ends of a longitudinal connector such that the opposite ends of the longitudinal connector span a distance between two components of the patient's anatomy;
positioning a first clamp assembly affixed to the first end of the longitudinal connector;
coupling the first clamp assembly to a first component of the patient's anatomy by inserting a first fastener through the first clamp assembly;
adjusting relative positions of the longitudinal connector and the first clamp assembly;
fixing the first clamp assembly to the first component of the patient's anatomy to thereby fix relative positions of the longitudinal connector and the first clamp assembly; and
securing the second end of the longitudinal connector to a second component of the patient's anatomy.

9. An anatomical bracing device for securing relative positions of a patient's anatomy, comprising:
a longitudinal connector having a proximal end and at least one distal end;
at least two clamp assemblies, affixed to each end of the longitudinal connector; and
at least one fastener extending through each of the clamp assemblies; wherein
the bracing device is configured such that securing the fasteners to the patient's anatomy both secures the longitudinal connector to the patient's anatomy and fixes relative positions of the longitudinal connector and clamp assemblies relative to each other.
